# EUROPEAN PATENT APPLICATION

(11) **EP 4 331 689 A1**
(43) Date of publication of application: **06.03.2024**
(21) Application number: 22192864.1
(22) Date of filing: 30.08.2022
(51) Int. Cl.: A62B 7/14, A61B 5/08, A61B 5/00

(54) **AIRCRAFT EMERGENCY OXYGEN SUPPLY SYSTEM, AIRCRAFT COMPRISING SUCH AN EMERGENCY OXYGEN SUPPLY SYSTEM, AND METHOD OF OPERATING AN AIRCRAFT EMERGENCY OXYGEN SUPPLY SYSTEM**

(71) Applicant: B/E Aerospace Systems GmbH, 23560 Lübeck (DE)
(72) Inventor: Degenhardt, Detlev, 23617 Stockelsdorf (DE)
(74) Representative: Schmitt-Nilson Schraud Waibel Wohlfrom Patentanwälte Partnerschaft mbB

(57) **Abstract**

An aircraft emergency oxygen supply system (2) comprises a passenger oxygen mask (12) for supplying oxygen to a passenger wearing the passenger oxygen mask (12); an oxygen source (20) for supplying oxygen to the passenger oxygen mask (12); a breath sensor (36) for detecting breaths (30) of the passenger wearing the passenger oxygen mask (12); and a controller (18) for controlling the supply of oxygen from the oxygen source (20) to the passenger oxygen mask. The controller (18) is configured for operating in a normal breathing mode, which includes supplying a normal breathing amount of oxygen (V_{N}) from the oxygen source (20) to the passenger oxygen mask (12) at every breath (30) of the passenger wearing the passenger oxygen mask (12); deriving a breathing frequency indicator from a plurality of successive breaths (30) of the passenger; and switching into a fast or into a low breathing mode if the breathing frequency indicator indicates a breathing frequency above or below predefined frequency thresholds. Operating in the fast or slow breathing mode includes supplying a fast or slow breathing amount of oxygen (V_{F}, V_{S}), which differs from the normal breathing amount of oxygen (VN), from the oxygen source (20) to the passenger oxygen mask (12) at every breath (30) of the passenger.

## Description

The present invention is in the field of aircraft equipment. The present invention is in particular in the field of emergency equipment for aircraft. Further in particular, the present invention is in the field of aircraft emergency oxygen supply systems in a passenger aircraft, aircraft comprising such emergency oxygen supply systems, and methods of operating such an aircraft emergency oxygen supply system.

Modern aircraft comprising a pressurized cabin are equipped with emergency passenger oxygen masks, which are deployed for the passengers in case of a loss of cabin pressure during flight in high altitudes. The passenger oxygen masks may be part of self-sustained aircraft emergency oxygen supply systems, including oxygen sources that provide gaseous oxygen to the passengers, in case cabin pressure is lost.

In case of pressure loss within the passenger cabin, the passenger oxygen masks are provided to the passengers, which may apply the passenger oxygen masks to their faces for breathing via the passenger oxygen masks. In modern aircraft emergency oxygen supply systems, the breaths of the passengers wearing the passenger oxygen masks may be detected, and a predefined amount of gaseous oxygen may be supplied to each passenger oxygen mask every time a breath of a passenger wearing the passenger oxygen mask is detected.

The passenger oxygen mask may comprise an ambient air valve, which allows ambient are to enter into the passenger oxygen mask in a controlled manner. The ambient air, which enters into the passenger oxygen mask via the ambient air valve, mixes with the gaseous oxygen, which is supplied by the oxygen source, for providing a physiological gas mixture, which comprises enough oxygen for providing a sufficient supply of oxygen to a passenger inhaling the gas mixture.

The predefined amount of oxygen, which is supplied to each passenger oxygen mask, has been set in advance based on typical human breathing needs, such as a typical human breath volume and a typical human oxygen demand.

The provision of oxygen via such aircraft emergency oxygen supply systems is not always satisfactory. In particular, such aircraft emergency oxygen supply systems may run out of oxygen early or supply not enough oxygen to the passengers in certain operating scenarios.

Accordingly, it would be beneficial to provide an improved aircraft emergency oxygen supply system and an improved method of supplying oxygen to a passenger oxygen mask in an aircraft. In particular, it would be beneficial to provide an aircraft emergency oxygen supply system and a method of supplying oxygen to a passenger oxygen mask in an aircraft which have a lower risk of running out of oxygen early and/or which have a lower risk of supplying insufficient oxygen to the passenger in case of a pressure loss within the aircraft passenger cabin.

Exemplary embodiments of the invention include an aircraft emergency oxygen supply system that comprises a passenger oxygen mask for supplying gaseous oxygen to a passenger wearing the passenger oxygen mask; an oxygen source for supplying oxygen to the passenger oxygen mask; a breath sensor for detecting breaths of the passenger wearing the passenger oxygen mask; and a controller for controlling the supply of oxygen from the oxygen source to the passenger oxygen mask.

The oxygen source may be an oxygen storage device, such as a gas bottle, filled with compressed oxygen gas, or a chemical oxygen generator, which, after being activated, starts a chemical reaction generating oxygen gas. The oxygen source may be equipped with a mechanical starter or with a pyroelectric starter, which are configured for starting the supply of oxygen from the oxygen source in response to receiving a start signal, in particular an electrical start signal.

The breath sensor may be configured for detecting a negative pressure, which is generated within the passenger oxygen mask each time the passenger wearing the passenger oxygen mask inhales.

The controller is configured for selectively operating in a normal breathing mode and for deriving a breathing frequency indicator from a plurality of successive breaths of the passenger. The controller is further configured for switching into a slow breathing mode, if the breathing frequency indicator indicates that the breathing frequency is below a predefined lower frequency threshold, and/or for switching into a fast breathing mode, if the breathing frequency indicator indicates that the breathing frequency is above a predefined upper frequency threshold.

Exemplary embodiments of the invention also include a method of supplying oxygen to a passenger oxygen mask in an aircraft, wherein the method includes: detecting breaths of a passenger wearing the passenger oxygen mask; operating in a normal breathing mode, which includes supplying a normal breathing amount of gaseous oxygen from the oxygen source to the passenger oxygen mask at, in particular at the beginning of, every breath of the passenger wearing the passenger oxygen mask; and deriving a breathing frequency indicator from a plurality of successive detected breaths of the passenger. The method further includes switching into a slow breathing mode, if the breathing frequency indicator indicates that the breathing frequency is below a predefined lower frequency threshold, and/or switching into a fast breathing mode, if the breathing frequency indicator indicates a breathing frequency above a predefined upper frequency threshold.

Operating in the slow breathing mode includes supplying a slow breathing amount of oxygen, which is larger than the normal breathing amount of oxygen, from the oxygen source to the passenger oxygen mask at, in particular at the beginning of, every breath of the passenger. Being provided to the passenger per breath, the slow breathing amount of oxygen may also be referred to as slow breathing bolus amount of oxygen. Similarly, the normal breathing amount of oxygen may also be referred to as normal breathing bolus amount of oxygen.

Operating in the fast breathing mode includes supplying a fast breathing amount of oxygen, which is smaller than the normal breathing amount of oxygen, from the oxygen source to the passenger oxygen mask at, in particular at the beginning of, every breath of the passenger. Being provided to the passenger per breath, the fast breathing amount of oxygen may also be referred to as fast breathing bolus amount of oxygen.

The normal / slow / fast breathing amount of oxygen may be provided in any suitable way. For example, it is possible that the desired amount of oxygen is provided via a suitable opening time of a controlled valve. In an exemplary embodiment, the aircraft emergency oxygen supply system may comprise an oxygen tank, such as an oxygen bottle and a pressure reduction valve, with the pressure reduction valve reducing the oxygen pressure in the oxygen bottle to a working pressure within a working oxygen reservoir. The working pressure in the working oxygen reservoir may not be constant. It may in particular vary depending on the momentary oxygen pressure in the oxygen bottle and depending on the momentary downstream consumption of oxygen. The one or more passenger oxygen masks may be coupled to the working oxygen reservoir via a respective number of controlled valves. Each of the controlled valves may be provided with a critical nozzle. By providing respective combinations of a controlled valve and a critical nozzle, it is possible to provide an operating environment where the oxygen flow through the controlled valve only depends on the working pressure. By sensing the working pressure and taking into account the level of the working pressure, the opening times of the controlled valves can be chosen to provide the desired normal / slow / fast breathing amount of oxygen. It is understood that such as system set-up is exemplary only and that other system set-ups are possible as well.

Detecting breaths of a passenger wearing the passenger oxygen mask may include detecting a negative pressure, which is generated within the passenger oxygen mask, each time the passenger wearing the passenger oxygen mask inhales.

The term breathing frequency indicator includes all kinds of parameters that contain information suitable for indicating a breathing frequency. For example, the breathing frequency indicator may be a breathing frequency value.

If the breathing frequency indicator is a breathing frequency value, the condition of the breathing frequency indicator indicating a breathing frequency above a predefined upper frequency threshold may be implemented as a determination whether the breathing frequency value is larger than the upper predefined upper frequency threshold.

Similarly, the condition of the breathing frequency indicator indicating a breathing frequency below a predefined lower frequency threshold may be implemented as a determination whether the breathing frequency value is smaller than the predefined lower frequency threshold.

As another example, the breathing frequency indicator may be a time period value between successive breaths of the passenger. In this case, the condition of the breathing frequency indicator indicating a breathing frequency above a predefined upper frequency threshold may be implemented as a determination whether the time period value is below a predefined lower duration threshold, which corresponds to the predefined upper frequency threshold. Similarly, the condition of the breathing frequency indicator indicating a breathing frequency below a predefined lower frequency threshold may be implemented as a determination whether the time period value is above a predefined upper duration threshold, which corresponds to the predefined lower frequency threshold.

The time period between successive breaths includes the time period for inhaling, the time period for exhaling, and potential pauses between inhaling and exhaling. In the following, the time period between successive breaths is also referred to as breath duration.

Exemplary embodiments of the invention also include a passenger aircraft, in particular an airplane or a helicopter, which comprises at least one aircraft emergency oxygen supply system according to an exemplary embodiment of the invention.

According to an exemplary embodiment of the invention, a normal breathing amount of oxygen is supplied from the oxygen source to the passenger oxygen mask at every breath of the passenger wearing the passenger oxygen mask, in particular at the beginning of every breath of the passenger, when the controller operates in the normal breathing mode.

A fast breathing amount of oxygen is supplied from the oxygen source to the passenger oxygen mask at every breath of the passenger, in particular at the beginning of every breath of the passenger, when the controller operates in the fast breathing mode. The fast breathing amount of oxygen is smaller than the normal breathing amount of oxygen. The fast breathing amount of oxygen is supplied to the passenger per inhalation. It may also be referred to as fast breathing bolus amount of oxygen.

In consequence, the amount of gaseous oxygen, which is supplied to the passenger oxygen mask at every breath of the passenger is reduced, when it has been detected that the passenger wearing the passenger oxygen mask is breathing faster, i.e. with a higher frequency / with shorter breath durations, than a predefined usual breathing frequency. Since, in the fast breathing mode, the passenger is breathing more often and the smaller fast breathing amount of gaseous oxygen is supplied to the passenger more frequently, the overall amount of oxygen supplied to the passenger is still appropriate, i.e. the passenger is still supplied with sufficient oxygen when the aircraft emergency oxygen supply system is operated in the fast breathing mode.

By reducing the amount of oxygen, which is supplied to the passenger oxygen mask at every breath, a fast depletion of the oxygen source may be prevented, even if the passenger is breathing considerably faster, i.e. with a considerably higher frequency, than a typical human adult. Exemplary embodiments of the invention may in particular allow to prevent that the oxygen source runs out of oxygen before the aircraft is able to descend to a lower altitude, at which the passengers are able to absorb sufficient oxygen from the air within the passenger cabin without being supplied with additional oxygen from the oxygen source.

The adaptation of the amount of oxygen per breath may be particularly beneficial in case small children or infants use the passenger oxygen mask, because small children or infants usually breathe with a higher frequency than adults, but do not require a larger total amount of oxygen. As compared to previous approaches, where the aircraft emergency oxygen supply system provides the same amount of oxygen to a fast breathing child and to a slower breathing adult, oxygen may be conserved and the risk of running out of oxygen early can be reduced. The amount of oxygen may be more targeted to an infant / child, and less oxygen may be wasted per breath of the infant / child. Situations may be prevented where the oxygen source, used by an infant / child, runs out, even before the aircraft is able to descend to a sufficiently low altitude, at which the passengers are able to breath ambient air from the passenger cabin without being supplied with additional oxygen from the aircraft emergency oxygen supply system.

The predefined upper frequency threshold, at which the controller switches from the normal breathing mode into the fast breathing mode, may be in the range from between 15 breaths per minute to 25 breaths per minute. When expressed as a corresponding lower breath duration threshold, the threshold may be in the range from between 2.4 seconds and 4 seconds. In other words, the controller may switch from the normal breathing mode to the fast breathing mode, if the detected breathing frequency of the passenger is above the predefined upper frequency threshold, which has been set to a value in the range of between 15 breaths per minute and 25 breaths per minute. While the comparison may be carried out for frequency values, it is possible to make the determination in the breath duration domain, as explained above. In particular, the controller may switch from the normal breathing mode to the fast breathing mode, if the detected breath duration of the passenger is below a given lower threshold, which has been set to a value in the range of between 2.4 seconds and 4 seconds.

When the controller operates in the slow breathing mode, a slow breathing amount of oxygen is supplied from the oxygen source to the passenger oxygen mask at every breath of the passenger, in particular at the beginning of every breath of the passenger. The slow breathing amount of oxygen is larger than the normal breathing amount of oxygen. The slow breathing amount of oxygen is supplied to the passenger per inhalation. It may also be referred to as slow breathing bolus amount of oxygen.

In consequence, the amount of gaseous oxygen, which is supplied to the passenger oxygen mask at every breath of the passenger is increased, when it has been detected that the passenger wearing the passenger oxygen mask is breathing slower, i.e. with a lower frequency / with larger breath durations, than a predefined usual breathing frequency. Since the passenger is breathing more rarely, but the larger slow breathing amount of gaseous oxygen is supplied to the passenger per breath, when the controller operates in the slow breathing mode, the overall amount of oxygen supplied to the passenger is still appropriate, i.e. the passenger is still supplied with sufficient oxygen for staying alive and awake when the aircraft emergency oxygen supply system is operated in the slow breathing mode.

By increasing the amount of oxygen, which is supplied to the passenger oxygen mask at every breath in the slow breathing mode, it is prevented that an insufficient amount of oxygen is supplied to and inhaled by a passenger who breathes slower, i.e. with a lower frequency, but with deeper breaths than an average passenger.

The predefined lower frequency threshold, at which the controller switches from the normal breathing mode into the slow breathing mode, may be in the range from between 5 breaths per minute to 15 breaths per minute, it may, in particular, be in the range from between 8 breaths per minute to 10 breaths per minute. When expressed as a corresponding upper breath duration threshold, the threshold may be in the range from between 4 seconds and 12 seconds, in particular in the range from between 6 seconds and 7.5 seconds.

In other words, the controller may switch from the normal breathing mode to the slow breathing mode, if the detected breathing frequency of the passenger is below the predefined lower frequency threshold, which has been set to a value in the range of between 5 breaths per minute and 15 breaths per minute. While the comparison may be carried out for frequency values, it is possible to make the determination in the breath duration domain, as explained above. In particular, the controller may switch from the normal breathing mode to the slow breathing mode, if the detected breath duration of the passenger is above a given upper threshold, which has been set to a value in the range of between 4 seconds and 12 seconds.

The predefined upper frequency threshold and/or the predefined lower frequency threshold may be fixed for all operating scenarios or may be dependent on one or more operating parameters. For example, the predefined upper frequency threshold and/or the predefined lower frequency threshold may be dependent on the ambient air pressure, which may be expressed as the aircraft altitude. In a particular exemplary embodiment, the predefined upper frequency threshold may be set to a value of 20 breaths per minute, in case the altitude is at or above 35.000 feet, and may be set to a value of 15 breaths per minute, in case the altitude is below 35.000 feet. Such an altitude-dependent setting of the predefined upper frequency threshold may be an additional safeguard to ensure that passengers receive sufficient oxygen at high altitudes, i.e. in very critical situations. The predefined upper frequency threshold and/or the predefined lower frequency threshold may be adapted during the emergency descent in accordance with such a set-up.

In order to prevent the controller from switching into the fast breathing mode right after the start of the aircraft emergency oxygen supply system, the controller may be configured not to switch into the fast breathing mode for the first 2 to 20 breaths of the passenger, in particular for the first 5 to 15 breaths of the passenger, after the supply of oxygen to the passenger mask has been activated. This may allow the breathing frequency of the passenger to settle somewhat before determining whether the passenger keeps breathing faster than usual, in which case the aircraft emergency oxygen supply system is switched into the fast breathing mode.

In order to prevent the controller from switching into the fast breathing mode or into the slow breathing mode in response to a single fast or slow breath of the passenger, the controller may be configured for switching from the normal breathing mode into the fast breathing mode or into the slow breathing mode only after a fast or slow breathing of the passenger has been detected for a predefined number of successive breaths, for example for 2 to 15 successive breaths, in particular 2 to 5 successive breaths.

In an embodiment, the controller is configured for determining the breathing frequency indicator from an average of a plurality of successive breath durations and for switching into the fast breathing mode only if said average indicates a fast breathing of the passenger, i.e. if an average breathing frequency is above the predefined upper frequency threshold or if an average breath duration is below a predefined lower breath duration threshold. Similarly, the controller may be configured for determining the breathing frequency indicator from an average of a plurality of successive breath durations and may be configured for switching into the slow breathing mode only if said average indicates a slow breathing of the passenger, i.e. if an average breathing frequency is below the predefined lower frequency threshold or if an average breath duration is above a predefined upper breath duration threshold.

Both the average breathing frequency and the average breath duration are possible implementations of the breathing frequency indicator. When determining the average, it is an option to pre-process the data set, such as by eliminating outliers, e.g. via eliminating the highest value and the lowest value, before determining the average. Such pre-processing is considered to be encompassed by the terminology of averaging the plurality of breath durations.

The average may be the average over between 5 and 30 detected breaths of the passenger, in particular over between 5 and 20 detected breaths of the passenger. In particular, the average may by the average over the last 5, 10, 15 or 20 detected breaths of the passenger. In order to achieve a high responsiveness of the aircraft emergency oxygen supply system and not to wait for the given numbers of detected breaths before reacting, the aircraft emergency oxygen supply system may operate with an assumed breathing history at start-up. For example, it is possible that the aircraft emergency oxygen supply system, at start-up, assumes a breathing history of 5, 10, 15 or 20 breaths of the passenger with a set breathing duration, such as a breathing duration of 4 seconds, which corresponds to a breathing frequency of 15 breaths per minute. In this way, the controller may, right from the start, determine the breathing frequency indicator from an average of a plurality of successive breath durations, while being able to provide a desired switching after a small number of breaths, such as 2 or 3 breaths, already.

The average may by an arithmetic average or a geometric average.

In an embodiment, the controller is configured for repeatedly determining the breathing frequency indicator on the basis of a moving average within a succession of breath durations of a succession of the most recent breaths of the passenger. Every average value of the moving average may be an average of between 10 and 20 breath durations, in particular an average of between 12 and 18 breath durations, further in particular an average of 15 breath durations.

In an embodiment, the controller is configured for applying a Kalman-Filter for damping accidental oscillations of the breathing frequency indicator.

In an embodiment, the aircraft emergency oxygen supply system comprises or is coupled with at least one air pressure sensor, which is configured for detecting an air pressure in the environment of the aircraft emergency oxygen supply system. The air pressure sensor may in particular be configured for detecting the air pressure within the aircraft passenger cabin, in which the aircraft emergency oxygen supply system has been installed.

In such an embodiment, the controller may be configured for setting the amount of oxygen, which is supplied to the passenger oxygen mask, as a function of the detected air pressure. This may be done for the normal breathing amount of oxygen, which is supplied in the normal breathing mode. Setting the amount of oxygen, which is supplied to the passenger oxygen mask, as a function of the detected air pressure may also be done for the fast breathing amount of oxygen, which is supplied when the aircraft emergency oxygen supply system is operated in the fast breathing mode, and/or for the slow breathing amount of oxygen, which is supplied when the aircraft emergency oxygen supply system is operated in the slow breathing mode.

Such an embodiment of the aircraft emergency oxygen supply system may allow for optimizing the amount of oxygen, which is supplied to the passenger oxygen mask, as a function of the air pressure within the passenger cabin.

In particular, when the air pressure within the passenger cabin is very low due to the aircraft flying at very high altitudes, a relatively large amount of oxygen may be supplied to the passenger oxygen mask at every breath of the passenger, in order to ensure that the passenger is supplied with sufficient oxygen.

On the other hand, when the air pressure within the passenger cabin is higher, because the aircraft is flying at a lower altitude, less oxygen needs to be supplied to the passenger. Thus, the amount of oxygen, which is supplied to the passenger oxygen mask at every breath of the passenger, may be reduced in order to prolong the maximum operating time of the oxygen source.

The amount of oxygen, which is supplied from the oxygen source to the passenger oxygen mask at every breath of the passenger, may be specified as a function of the air pressure, which has been detected within the passenger cabin. Different functions may be employed in the normal breathing mode, in the slow breathing mode, and in the fast breathing mode, respectively. Each function may be given as an analytic / arithmetic function or in any other suitable manner.

Alternatively, the different amounts of oxygen, which are supplied from the oxygen source to the passenger oxygen mask at every breath of the passenger, may be given by discrete values. A specific amount of oxygen, which is supplied to the passenger oxygen mask, may be assigned to at least one of a plurality of ranges of air pressure detected within the passenger cabin. The borders of the ranges of air pressure and the discrete values assigned to the different ranges of air pressure may differ between the normal breathing mode, the slow breathing mode, and the fast breathing mode. Such discrete values and their association to air pressure ranges may be given in the form of a look-up table or in any other suitable form.

It is also possible that the amount of oxygen, which is supplied from the oxygen source to the passenger oxygen mask, is given by discrete values in at least one range of detected air pressures, and that the amount of oxygen is given by at least one function in at least one other range of air pressures, as detected by the pressure sensor.

It is also possible that the amount of oxygen, which is supplied from the oxygen source to the passenger oxygen mask, is given by discrete values in at least one of the normal breathing mode, the slow breathing mode, and the fast breathing mode, and that the amount of oxygen is given by at least one function in at least one other of the normal breathing mode, the slow breathing mode, and the fast breathing mode.

In an embodiment, the aircraft emergency oxygen supply system comprises or is coupled to at least one temperature sensor, which is configured for detecting the temperature in the environment of the aircraft emergency oxygen supply system, in particular the temperature within the aircraft passenger cabin.

In such an embodiment, the controller may be configured for setting the amount of of oxygen, which is supplied to the passenger oxygen mask at every breath of the passenger, as a function of the detected temperature. This may apply to the normal breathing amount of oxygen, which is provided in the normal breathing mode. This may also apply to the slow breathing amount of oxygen, which is provided in the slow breathing mode, and/or to the fast breathing amount of oxygen, which is provided in the fast breathing mode.

The amount of oxygen, which is supplied from the oxygen source to the passenger oxygen mask at every breath of the passenger, may be defined as a function of the temperature, which has been detected within the passenger cabin. Different functions may be employed in the normal breathing mode, in the slow breathing mode, and in the fast breathing mode, respectively. Each function may be given as an analytic / arithmetic function or in any other suitable manner.

Alternatively, the amounts of oxygen, which are supplied from the oxygen source to the passenger oxygen mask at every breath of the passenger, may be defined by discrete values. A specific amount of oxygen, which is to be supplied to the passenger oxygen mask, may be assigned to each of a plurality of ranges of detected temperatures. The borders of the ranges of temperature and the discrete values assigned to the different ranges of temperature may differ in the normal breathing mode, in the slow breathing mode, and the in fast breathing mode, respectively. Such discrete values and their association to temperature ranges may be given in the form of a look-up table or in any other suitable form.

It is also possible that the amount of oxygen, which is supplied from the oxygen source to the passenger oxygen mask, is given by discrete values in at least one range of detected temperatures, and that the amount of oxygen is given by at least one function in at least one other range of detected temperatures.

In order to compensate for a low supply of oxygen to the passenger during an initial time period between the loss of cabin pressure and the supply of oxygen from the oxygen source to the passenger via the passenger oxygen mask, a maximum amount of oxygen may be supplied to the passenger oxygen mask right after the start of the aircraft emergency oxygen supply system, for example for the first 15 breaths taken by the passenger.

In other words, right after the start of the aircraft emergency oxygen supply system, the amount of oxygen, which is supplied to the passenger oxygen mask at each breath of the passenger, may be set to a maximum value, which is independent of the air pressure within the passenger cabin.

Later, for example after 15 breaths have been taken by the passenger, the amount of oxygen, which is supplied with each oxygen pulse from the oxygen source to the passenger oxygen mask, may be adjusted as a function of the air pressure within the passenger cabin, as is has been described before.

The predefined lower frequency threshold and/or the predefined upper breath duration threshold, at which the controller switches from the normal breathing mode into the slow breathing mode, may depend on the detected air pressure and/or on the detected temperature.

Similarly, the predefined upper frequency threshold and/or the predefined lower breath duration threshold, at which the controller switches from the normal breathing mode into the fast breathing mode, may depend on the detected air pressure and/or on the detected temperature as well.

In an embodiment, the amount of oxygen, which is supplied from the oxygen source to the passenger oxygen mask, is a function of the breathing frequency indicator, e.g. a function of the detected breathing frequency or the detected breath duration. In other words, within the normal breathing mode, within the slow breathing mode, and/or within the fast breathing mode, a granular adjustment of the normal breathing amount of oxygen, of the slow breathing amount of oxygen, and/or of the fast breathing amount of oxygen may take place. In yet other words, the normal breathing amount of oxygen, the slow breathing amount of oxygen, and/or the fast breathing amount of oxygen may be more than three set values for the normal breathing mode, the slow breathing mode, and the fast breathing mode.

The amount of oxygen, which is supplied from the oxygen source to the passenger oxygen mask, may be reduced if the detected breathing frequency increases and/ or the detected breath duration decreases, and the amount of oxygen, which is supplied from the oxygen source to the passenger oxygen mask, may be increased if the detected breathing frequency decreases and/or the detected breath duration increases.

The supplied amount of oxygen may in particular be proportional to the detected breath duration and/or inversely proportional to the detected breathing frequency. Alternatively, the supplied amount of oxygen may be a step function of the detected breathing frequency or the detected breath duration.

In an embodiment, this may apply to one of the normal breathing mode, the slow breathing mode, and the fast breathing mod. In further embodiments, this may apply to any two or all three of these breathing modes.

In other words, when the breathing frequency indicator indicates that the current breathing frequency is within a defined normal breathing frequency range, so that the controller operates in the normal breathing mode, the supplied amount of oxygen may be adjusted within the normal breathing range based on the breathing frequency indicator according to a normal breathing mode adjustment function.

When the breathing frequency indicator indicates that the current breathing frequency is within the slow breathing range, so that the controller operates in the slow breathing mode, the supplied amount of oxygen may be adjusted within the slow breathing range based on the breathing frequency indicator according to a slow breathing mode adjustment function.

Similarly, when the breathing frequency indicator indicates that the current breathing frequency is within the fast breathing range, so that the controller operates in the fast breathing mode, the supplied amount of oxygen may be adjusted within the fast breathing range based on the breathing frequency indicator according to a fast breathing mode adjustment function.

The normal breathing mode adjustment function, the slow breathing mode adjustment function, and the fast breathing mode adjustment function may be implemented as analytic functions or as step functions, which may be represented by respective look-up tables, or in any other suitable manner.

Adjusting the amount of oxygen supplied to the passenger oxygen mask based on the derived breathing frequency indicator may allow for an even better adjustment of the amount of oxygen supplied to the passenger wearing the passenger oxygen mask.

The amount of oxygen, which is supplied to the passenger oxygen mask at every breath, may be adjusted as a function of the breathing frequency indicator in a range of between 6 breaths per minute to 60 breaths per minute. This may also be expressed as adjusting the amount of oxygen for breath durations which are in the range from between 1 second to 10 seconds.

In an embodiment, the amount of oxygen, which is supplied from the oxygen source to the passenger oxygen mask, may be independent of the breathing frequency indicator in at least one of the normal breathing mode, the slow breathing mode, and the fast breathing mode. In other words, the amount of oxygen, which is supplied from the oxygen source to the passenger oxygen mask, may be unad-justed / fixed in the normal breathing mode, in the slow breathing mode, and/or in the fast breathing mode. Such an embodiment, in which the amount of oxygen, which is supplied to the passenger oxygen mask, is fixed / not adjusted, may result in a more simple implementation of the controller.

In an embodiment, the controller is configured for switching into an enforced pulse mode if no breath of a passenger has been detected by the breath sensor for a predefined period of time, which is also referred to herein as breath enforce time. The breath enforce time may be in the range of between 5 seconds and 15 seconds, the breath enforce time may in particular be in the range of between 9 seconds and 11 seconds, such as 10 seconds.

After the controller has been switched into the enforced pulse mode, the controller may be configured to supply predefined amounts of oxygen, which may be called enforced pulse amounts of oxygen, from the oxygen source to the passenger oxygen mask at predefined enforced pulse mode time intervals. In consequence, the supply of oxygen to the passenger oxygen mask is independent of the detection of passenger breaths by the breath sensor, when the controller is operating in the enforced pulse mode.

Operating the controller in the enforced pulse mode may ensure that the passenger is supplied with sufficient oxygen even if his/her breaths are not detected by the breath sensor. It is possible that the breaths of the passenger are not detected by the breath sensor because the passenger is not breathing strong enough and/or because the passenger oxygen mask is not sitting tight enough on the passenger's face, thereby allowing ambient air to flow uncontrolled into the passenger oxygen mask. In such as situation, the negative pressure, which is generated within the passenger oxygen mask, when the passenger inhales, is not sufficiently large for being detected as a breath by the breath sensor.

In an embodiment, the enforced pulse amount of oxygen is dependent on the ambient air pressure, i.e. the enforced pulse amount of oxygen may be a function of the air pressure detected by an air pressure sensor. As explained above, the ambient air pressure may be expressed as the aircraft altitude.

When operating in the enforced pulse mode, the controller may be configured to supply enforced pulse amounts of oxygen to the passenger oxygen mask with a frequency of 40 to 60 pulses per minute, in particular with a frequency of 45 to 55 pulses per minute, more particularly with a frequency of 50 pulses per minute.

In an embodiment, the aircraft emergency oxygen supply system comprises a plurality of passenger oxygen masks, and the controller is configured for individually controlling the supply of oxygen to each of the plurality of passenger oxygen masks. The aircraft emergency oxygen supply system may in particular comprise two, three, four, five, six or more passenger oxygen masks.

An aircraft emergency oxygen supply system comprising a plurality of passenger oxygen masks, which are controlled by a single controller, allows for an efficient use of the controller. In consequence, the costs of an aircraft emergency oxygen supply system, comprising a given number of oxygen masks, may be reduced compared to a configuration in which an individual controller is provided for each oxygen mask.

The additional features, modifications, and effects, as described herein with respect to the aircraft emergency oxygen supply system, apply to the method of supplying oxygen to a passenger oxygen mask in an aircraft, as described herein, in an analogous manner, and vice versa. Also, the additional features, modifications, and effects, as described herein with respect to the aircraft emergency oxygen supply system and/or the method of supplying oxygen to a passenger oxygen mask in an aircraft, apply to an aircraft in accordance with exemplary embodiments of the invention in an analogous manner.

Further exemplary embodiments of the invention are described in the following with respect to the accompanying drawings, wherein:
Figure 1 shows a schematic side view of an aircraft, in particular an air plane, according to an exemplary embodiment of the invention;
Figure 2 shows a perspective view of an aircraft emergency oxygen supply system according to an exemplary embodiment of the invention, with the passenger oxygen masks of the aircraft passenger oxygen supply module being shown in a deployed state;
Figure 3 shows a schematic block diagram of an aircraft emergency oxygen supply system according to an exemplary embodiment of the invention;
Figure 4 is a diagram that schematically illustrates the supply of bolus amounts of oxygen in the operation of an aircraft emergency oxygen supply system according to an exemplary embodiment of the invention in a normal breathing mode;
Figure 5 is a diagram that schematically illustrates the supply of bolus amounts of oxygen in the operation of an aircraft emergency oxygen supply system according to an exemplary embodiment of the invention in a fast breathing mode;
Figure 6 is a diagram that schematically illustrates the supply of bolus amounts of oxygen in the operation of an aircraft emergency oxygen supply system according to an exemplary embodiment of the invention in a slow breathing mode.

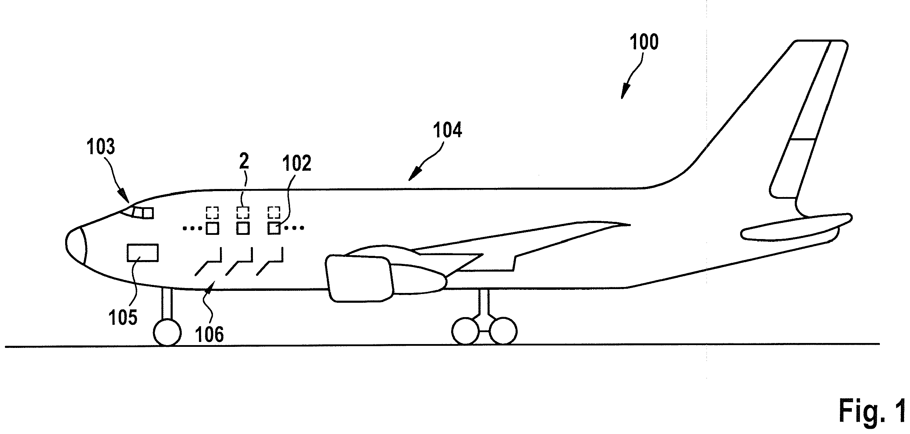

Figure 1 shows an aircraft 100, in particular an air plane, in accordance with an exemplary embodiment of the invention in a schematic side view. In the exemplary embodiment shown in Figure 1, the aircraft 100 is a large passenger air plane, comprising a cockpit 103 and a passenger cabin 104, housing a plurality of passenger seats 106. In general, the aircraft may be a commercial passenger air plane, a private air plane, a military aircraft, or a rotorcraft, such as a helicopter.

In an exemplary configuration, in which the aircraft 100 comprises six passenger seats 106 in every row, each row of passenger seats 106 may have two aircraft emergency oxygen supply systems 2 associated therewith, one aircraft emergency oxygen supply system 2 assigned to the passenger seats 106 on the left side of a center aisle and one aircraft emergency oxygen supply system 2 assigned to the passenger seats 106 on the right side of the center aisle.

For the exemplary embodiment of each row of passenger seats 106 having six seats, every aircraft emergency oxygen supply system 2 may include one oxygen source 20 and three or more passenger oxygen masks 12, coupled to the oxygen source 20. Such a set-up is schematically illustrated in Figure 1 via three exemplary passenger windows 102, each being associated with a row of passenger seats 106, and via three exemplary aircraft emergency oxygen supply systems 2, depicted in phantom due to their arrangement within the aircraft 100.

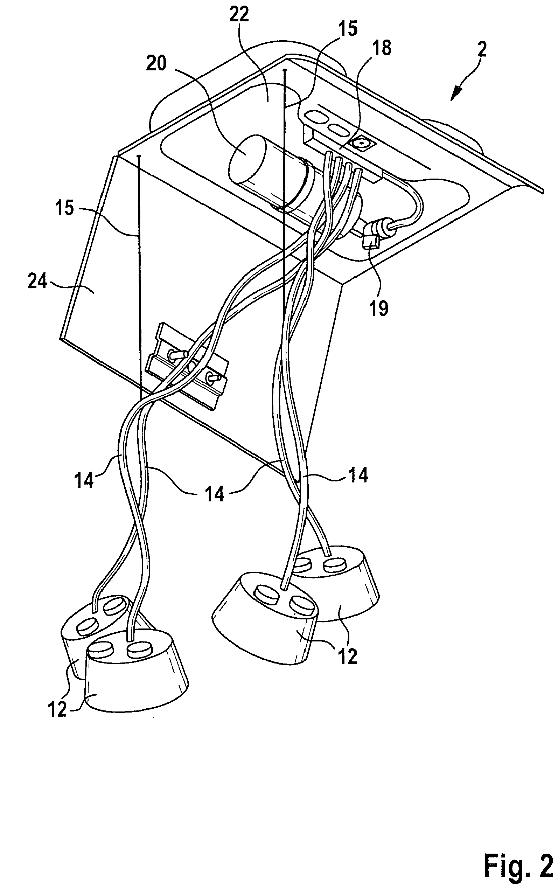

Figure 2 depicts an aircraft emergency oxygen supply system 2 in accordance with an exemplary embodiment of the invention, as it may be installed in the passenger cabin 104 of an aircraft 100. The aircraft emergency oxygen supply system 2 depicted in Figure 2 comprises an oxygen source 20 and four passenger oxygen masks 12. More or less than four passenger oxygen masks 12, such as two, three, five or six passenger oxygen masks, may be provided. Each passenger oxygen mask 12 is fluidly coupled with the oxygen source 20 by an oxygen hose 14, allowing the oxygen source 20 to supply gaseous oxygen via the oxygen hose 14 to the respective passenger oxygen mask 12.

During normal operation of the aircraft 100, the passenger oxygen masks 12 are stored within a compartment 22, and a movable door 24 of the aircraft emergency oxygen supply system 2 is in a closed position, in which it covers the oxygen source 20 and prevents the passenger oxygen masks 12 from dropping out of the compartment 22.

In case of a pressure loss within the passenger cabin 104, the movable door 24 opens, allowing the passenger oxygen masks 12 to drop out of the compartment 22, so that a passenger sitting below the compartment 22 may grab one of the passenger oxygen masks 12 and apply it to his face. The oxygen masks 12 may be held by mechanical links 15, such as cords or wires, in front of the passengers. The oxygen source 20 is equipped with a starter 19, which may be a mechanical starter a pyroelectric starter. Upon activation via an according signal, the starter 19 may start the release of oxygen from the oxygen source 20, such as via breaking a seal of the oxygen source 20.

The aircraft emergency oxygen supply system 2 comprises a controller 18, which is configured for controlling the supply of oxygen from the oxygen source 20 to the passenger oxygen masks 12.

The supply of oxygen from the oxygen source 20 to a passenger oxygen mask 12 is started, after a first breath has been taken by the passenger and said first breath has been detected by a breath sensor (not shown in Figure 2), which is arranged along the flow path between the oxygen source 20 and the respective passenger oxygen mask 12.

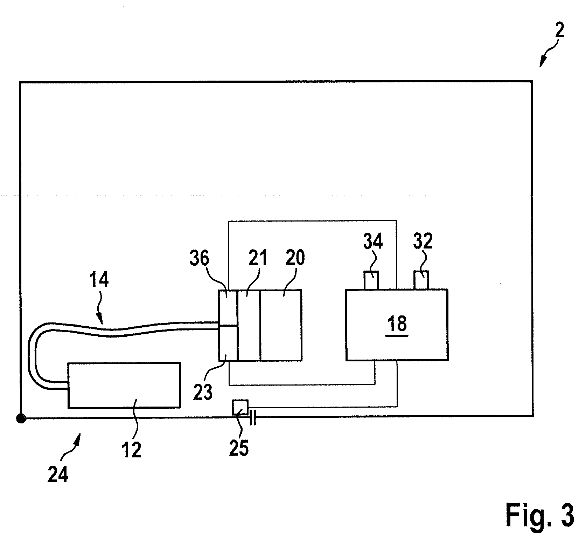

Figure 3 shows a schematic block diagram of an aircraft emergency oxygen supply system 2 in accordance with an exemplary embodiment of the invention.

For simplicity of illustration, only a single passenger oxygen mask 12, which is fluidly coupled to an oxygen source 20 by means of an oxygen hose 14, is depicted in Figure 3. In the exemplary embodiment of Figure 3, the oxygen source 20 is equipped with a pressure reduction valve and a working oxygen reservoir, jointly indicated at block 21. With the pressure reduction valve, the pressure of the oxygen from the oxygen source 20, which may be an oxygen bottle, is reduced to a working pressure for the oxygen in the working oxygen reservoir. Via an oxygen control device 23, which may in particular be a controlled valve, the oxygen from the working oxygen reservoir is supplied to the oxygen hose 24.

The skilled person understands that an aircraft emergency oxygen supply system 2, as it is depicted in Figure 3, may comprise more than one passenger oxygen mask 12 as well. A plurality of passenger oxygen masks 12 may be fluidly coupled to a common oxygen source 20, as it is shown in Figure 2. Alternatively, the aircraft emergency oxygen supply system 2 may comprise a plurality of oxygen sources 20. In particular, the aircraft emergency oxygen supply system 2 may comprise a separate oxygen source 20 for each passenger oxygen mask 12.

The aircraft emergency oxygen supply system 2 further comprises a controller 18, which is configured for controlling the operation of the aircraft emergency oxygen supply system 2.

The controller 18 is in particular configured for controlling an actuator 25, which is provided for unlocking and/or opening the movable door 24 of the aircraft emergency oxygen supply system 2, in order to provide passengers with access to the at least one passenger oxygen mask 12 of the aircraft emergency oxygen supply system 2.

The controller 18 is further configured for controlling the supply of oxygen from the oxygen source 20 to the passenger oxygen mask 12. In particular, the controller 18 is configured for controlling the oxygen control device 23 for effecting a controlled supply of oxygen to the passenger oxygen mask 12. In case the aircraft emergency oxygen supply system 2 comprises a plurality of passenger oxygen masks 12, an individual oxygen control device 23 is provided for each passenger oxygen mask 12, respectively, so that the controller 18 can control the oxygen flow to the passengers wearing the passenger oxygen masks 12 individually for each passenger oxygen mask 12.

The aircraft emergency oxygen supply system 2 comprises at least one breath sensor 36, which is configured for detecting the breaths of a passenger, who is wearing the passenger oxygen mask 12. In case the aircraft emergency oxygen supply system 2 comprises a plurality of passenger oxygen masks 12, an individual breath sensor 36 is provided for each passenger oxygen mask 12, respectively, so that the controller 18 receives information about the breaths of the passengers wearing the passenger oxygen masks 12 individually for each passenger oxygen mask 12.

The controller 18 is configured for deriving a breathing frequency indicator from the breaths of the passenger, which are detected by the breath sensor 36.

The breathing frequency indicator may represent a breathing frequency of successive breaths of the passenger. It is also possible that the breathing frequency indicator represents the lengths of time periods between successive breaths of the passenger. A time period between successive breaths includes the time period for inhaling, the time period for exhaling and potential pauses between inhaling and exhaling. In the following, the time period between successive breaths is also referred to as breath duration.

The controller 18 may be configured for determining the breathing frequency indicator from an average of a plurality of successive breath durations. The breathing frequency indicator may in particular be determined from a moving average within a succession of breath durations of a succession of breaths of the passenger. Every average value of the moving average may in particular be an average of the last 10 and 20 breath durations, further in particular an average of the last 15 breath durations.

Determining the breathing frequency indicator may also include the use of a Kalman-Filter for smoothening accidental oscillations of the breathing frequency indicator.

If the breathing frequency indicator represents a breathing frequency value or an average breathing frequency value of the passenger, the controller 18 operates in a normal breathing mode when said value is below a predefined upper threshold and above a predefined lower threshold. If the breathing frequency indicator is implemented as a breath duration value or an average value of successive breath durations, the controller 18 operates in the normal breathing mode if said value is above a predefined lower threshold and below a predefined upper threshold. In other words, the controller 18 operates in the normal breathing mode if the passenger wearing the passenger oxygen mask 12 does not breathe unusually fast or slow, but with a breathing frequency within a predefined "normal breathing frequency range", which is considered to correspond to normal breathing.

When the controller 18 operates in the normal breathing mode, an amount of oxygen, which is associated with the normal breathing mode, i.e. a normal breathing amount of oxygen V_{N}, is supplied from the oxygen source 20 to the passenger oxygen mask 12 at every breath of the passenger. The normal breathing amount of oxygen V_{N} may in particular be supplied to the passenger oxygen mask 12 at the beginning of each breath of the passenger, in order to allow the passenger to inhale the supplied amount of oxygen, which results in a sufficient supply of oxygen to the passenger.

As mentioned before, children usually breath faster than adults and inhale a smaller amount, i.e. volume, of air with each breath. Thus, if the aircraft emergency oxygen supply system 2 were to operate in the normal breathing mode for supplying oxygen to a child, oxygen pulses comprising the normal breathing amount of oxygen V_{N} would be supplied to the passenger oxygen mask 12 with a higher than normal frequency, but only a portion of the oxygen, supplied to the passenger oxygen mask 12 with each oxygen pulse, would be inhaled by the child. As a result, the oxygen supply in the oxygen source 20 would empty fast due to the high frequency of oxygen pulses, and a large portion of the supplied oxygen would be wasted, as it would not be inhaled by the child wearing the passenger oxygen mask 12. In consequence, there would be a risk that the oxygen source 20 would run out early, in particular before an aircraft 100 flying at a high altitude would be able to descend to a lower altitude, at which the passengers are able to inhale sufficient oxygen with the ambient air.

In order to avoid said waste of oxygen and to prevent the oxygen source 20 from running out early, the controller 18 of an aircraft emergency oxygen supply system 2 according to an exemplary embodiment of the invention is configured for switching into a fast breathing mode if the breathing frequency indicator indicates that the breathing frequency of the passenger wearing the passenger oxygen mask 12 is above a predefined upper frequency threshold.

The breathing frequency indicator indicating that the breathing frequency is above a predefined upper frequency threshold may be implemented by a breathing frequency value being above the predefined upper frequency threshold or by a breath duration value being below a predefined lower breath duration threshold, corresponding to the predefined upper frequency threshold.

The predefined upper frequency threshold, at which the controller switches from the normal breathing mode into the fast breathing mode, may be in the range from between 15 breaths per minute to 25 breaths per minute, in particular at 20 breaths per minute. Correspondingly, a predefined lower breath duration threshold, at which the controller switches from the normal breathing mode into the fast breathing mode, may be set to breath durations of between 2.4 seconds and 4 seconds, in particular to a breath duration of 3 seconds.

When the aircraft emergency oxygen supply system 2 is operated in the fast breathing mode, a fast breathing amount of oxygen VF, which is smaller than the normal breathing amount of oxygen V_{N}, is supplied from the oxygen source 20 to the passenger oxygen mask 12 at every breath of the passenger, in particular at the beginning of every breath of the passenger wearing the passenger oxygen mask 12.

As a result of reducing the amount of oxygen, which is supplied at each breath of the passenger from the oxygen source 20 to the passenger oxygen mask 12, the increased frequency of the oxygen pulses is at least partially compensated by a reduced amount (volume) of oxygen, which is supplied with each pulse. In consequence, the maximum operating time of the oxygen source 20 is extended.

The fast breathing amount of oxygen V_{F} may in particular be set such that the maximum operating time of the oxygen source 20 in the fast breathing mode is substantially the same as the maximum operating time in the normal breathing mode. It can also be said that the fast breathing amount of oxygen VF may be set such that a similar or substantially the same amount of oxygen per minute is supplied in the fast breathing mode and in the normal breathing mode.

The fast breathing amount of oxygen V_{F} may, for example, be between 25 % and 75 % of the normal breathing amount of oxygen V_{N}. The fast breathing amount of oxygen V_{F} may in particular be between 33 % and 66 % of the normal breathing amount of oxygen V_{N}. More particularly, the fast breathing amount of oxygen VF may be approximately 50 % of the normal breathing amount of oxygen V_{N}.

Similarly, the controller 18 of an aircraft emergency oxygen supply system 2 according to an exemplary embodiment of the invention may be configured for switching into a slow breathing mode if the breathing frequency indicator indicates that the breathing frequency of the passenger wearing the passenger oxygen mask 12 is below a predefined lower frequency threshold.

The breathing frequency indicator indicating that the breathing frequency is below a predefined lower frequency threshold may be implemented by a breathing frequency value being below the predefined lower frequency threshold or by a breath duration value being above a predefined upper breath duration threshold, which corresponds to the predefined lower frequency threshold.

The predefined lower frequency threshold, at which the controller switches from the normal breathing mode into the slow breathing mode, may be in the range from between 5 breaths per minute to 15 breaths per minute, in particular at 10 breaths per minute. Correspondingly, a predefined upper breath duration threshold, at which the controller switches from the normal breathing mode into the slow breathing mode, may be set to breath durations of between 4 seconds and 12 seconds, in particular to a breath duration of between 8 seconds and 10 seconds.

When the aircraft emergency oxygen supply system 2 is operated in the slow breathing mode, a slow breathing amount of oxygen V_{S}, which is larger than the normal breathing amount of oxygen V_{N}, is supplied from the oxygen source 20 to the passenger oxygen mask 12 at every breath of the passenger, in particular at the beginning of every breath of the passenger wearing the passenger oxygen mask 12.

As a result of increasing the amount of oxygen, which is supplied at each breath of the passenger from the oxygen source 20 to the passenger oxygen mask 12, the reduced frequency of the oxygen pulses is at least partially compensated by an increased amount (volume) of oxygen, which is supplied with each pulse. In consequence, a slow breathing passenger is supplied with a sufficient amount of oxygen as well.

The slow breathing amount of oxygen V_{S} may, for example, be between 125 % and 300 % of the normal breathing amount of oxygen V_{N}. The slow breathing amount of oxygen V_{S} may in particular be between 150 % and 250 % of the normal breathing amount of oxygen V_{N}. More particularly, the slow breathing amount of oxygen V_{S} may be approximately 200 % of the normal breathing amount of oxygen V_{N}.

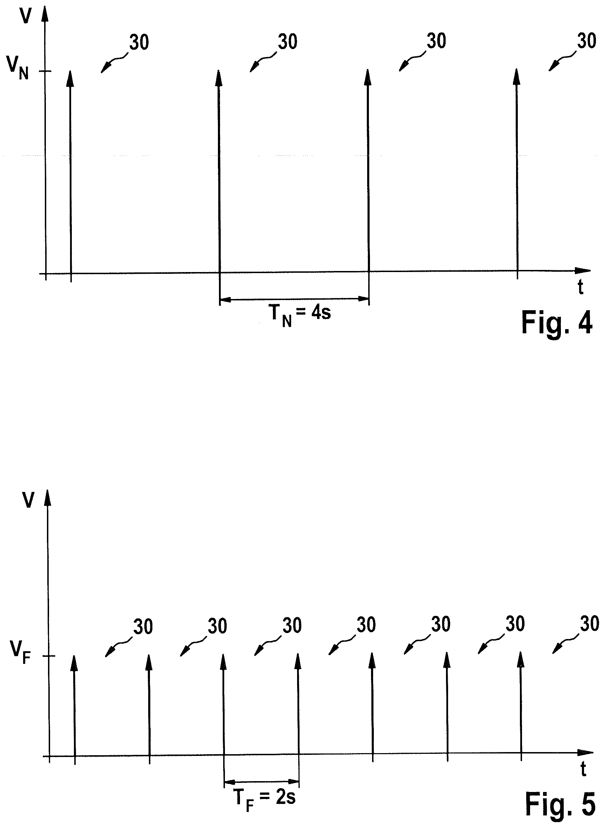

Figure 4 is a diagram that schematically illustrates the supply of bolus amounts of oxygen in the operation of the aircraft emergency oxygen supply system 2 of Figure 3 in the normal breathing mode.

In the example of the normal breathing mode, which is depicted in Figure 4, the breath duration, i.e. the length of the time intervals between successive oxygen pulses 30, is T_{N} = 4 s. This corresponds to an oxygen pulse frequency fN = 60 s/TN = 15 pulses per minute. The amount of oxygen, which is supplied to the passenger oxygen mask 12 with each pulse is the normal breathing amount of oxygen V_{N}.

Figure 5 shows a diagram that schematically illustrates the supply of bolus amounts of oxygen in the operation of the aircraft emergency oxygen supply system 2 of Figure 3 in the fast breathing mode.

In the example of the fast breathing mode, which is depicted in Figure 5, the breath duration, i.e. the exemplary length of the time intervals between successive oxygen pulses 30, is T_{F} = 2 s. This corresponds to an oxygen pulse frequency f_{F} = 60 s/T_{F} = 30 pulses per minute. The amount of oxygen, which is supplied to the passenger oxygen mask 12 with each the pulse is the fast breathing amount of oxygen VF.

In the exemplary configurations depicted in Figures 4 and 5, the breath duration in the normal breathing mode TN is twice as large as the breath duration in the fast breathing mode T_{F}, (TN = 2 TF), and correspondingly, the frequency in the normal breathing mode fN is half of the frequency in the fast breathing mode fF (f_{N} = 1/2 fF). The fast breathing amount of oxygen VF is half of the normal breathing amount of oxygen V_{N} (V_{F} = 1/2 VN). In this way, substantially the same amount of oxygen per minute may be provided to the passenger in the normal breathing mode and the fast breathing mode.

These ratios are, however, only exemplary and the ratios between the breath durations T_{N}, T_{F} , the breathing frequencies f_{N}, f_{F} and the amounts V_{N}, V_{F} of oxygen in the normal breathing mode and in the fast breathing mode may be different.

The fast breathing amount of oxygen V_{F} may, for example, be 25%, 33%, 66% or 75% of the normal breathing amount of oxygen V_{N} or may be any other amount in between.

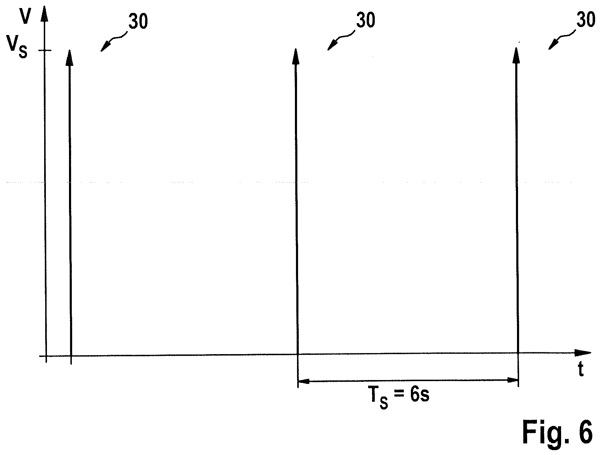

Figure 6 shows a diagram that schematically illustrates the supply of bolus amounts of oxygen in the operation of the aircraft emergency oxygen supply system 2 of Figure 3 in the slow breathing mode.

In the example of the slow breathing mode, which is depicted in Figure 6, the breath duration, i.e. the exemplary length of the time intervals between successive oxygen pulses 30, is T_{S} = 6 s. This corresponds to an oxygen pulse frequency fs = 60 s/T_{S} = 10 pulses per minute. The amount of oxygen, which is supplied to the passenger oxygen mask 12 with each the pulse is the slow breathing amount of oxygen V_{S}.

In the exemplary configurations depicted in Figures 4 and 6, the breath duration in the slow breathing mode T_{S} is 1.5 times the breath duration in the normal breathing mode T_{N}, (T_{S} = 1.5 T_{N}), and correspondingly, the frequency in the normal breathing mode fN is 1.5 times the frequency in the slow breathing mode f_{S} (f_{N} = 1.5 * f_{S}). The slow breathing amount of oxygen V_{S} is 150% of the normal breathing amount of oxygen V_{N} (V_{S} = 150% * V_{N}). In this way, substantially the same amount of oxygen per minute may be provided to the passenger in the normal breathing mode and the slow breathing mode.

These ratios are, however, only exemplary and the ratios between the breath durations T_{N}, T_{S} , the breathing frequencies f_{N}, f_{S} and the amounts V_{N}, V_{S} of oxygen in the normal breathing mode and in the slow breathing mode may be different.

The slow breathing amount of oxygen V_{S} may, for example, be 125%, 150%, 200%, 250% or 300% of the normal breathing amount of oxygen V_{N} or may be any other amount in between.

In the diagrams of Figures 4, 5, and 6, the normal, fast and slow breathing amounts of oxygen V_{N}, V_{S}, V_{F} are illustrated as pulses, in order to immediately demonstrate the different volumes. It is understood that the different volumes may be achieved in any suitable manner. For example, it is possible to provide a particular oxygen flow to the passenger oxygen mask 12 via a controlled valve, with the opening time of the controlled valve determining the supplied oxygen volume. In other words, the pulses in the normal, fast, and slow breathing modes may differ with respect to the lengths a particular oxygen flow is provided. It is also possible to vary the oxygen flow and/or to vary a combination of the oxygen flow and the length the oxygen flow is provided. All of these options are intended to be encompassed by the pulse representation of Figures 4, 5, and 6. It can also be said that the pulse representation of Figures 4, 5, and 6 is a representation of the oxygen bolus amount, irrespective of how the provision of the bolus amount is implemented.

The amount (volume) V_{N}, V_{S}, V_{F} of oxygen, which is supplied to the passenger oxygen mask in each pulse, may in particular be a function of the air pressure pₐᵢᵣ within the passenger cabin 104 of the aircraft 100.

In order to detect the current air pressure pₐᵢᵣ within the passenger cabin 104, the aircraft emergency oxygen supply system 2 may be employed with at least one air pressure sensor 32, as illustrated in Figure 3, which is configured for measuring the air pressure pₐᵢᵣ within the passenger cabin 104. Alternatively, a plurality of aircraft emergency oxygen supply systems 2 may receive information about the current air pressure pₐᵢᵣ within the passenger cabin 104 from a common air pressure sensor, which is coupled with the plurality of aircraft emergency oxygen supply systems 2.

In an embodiment, which includes an air pressure sensor 32, the controller 18 of the aircraft emergency oxygen supply system 2 may cause the oxygen source 20 to supply a larger amount of oxygen to the passenger oxygen mask 12 if the air pressure pₐᵢᵣ, which is detected within the passenger cabin 104, is low, since the aircraft 100 is flying at a high altitude, so that the air within the passenger cabin 14 is very thin, i.e. the air pressure pₐᵢᵣ within the passenger cabin 14 is low and a comparably small number of oxygen molecules are present in the ambient air per unit volume.

On the other hand, if the aircraft 100 is flying at a lower altitude closer to the ground, the ambient air pressure pₐᵢᵣ within the passenger cabin is higher and the ambient air within the passenger cabin 14 comprises a larger number of oxygen molecules per unit volume. In consequence, at lower altitudes, less additional oxygen needs to be provided by the aircraft emergency oxygen supply system 2 to the passenger, and thus, the amount of oxygen V_{N}, V_{S}, V_{F}, which is supplied from the oxygen source 20 to the passenger oxygen mask 12 may be reduced. Reducing the amount of oxygen V_{N}, V_{S}, V_{F}, which is supplied from the oxygen source 20 to the passenger oxygen mask 12, may increase the maximum operating time of the oxygen source 20.

The amount of oxygen V_{N}, V_{S}, V_{F}, which is supplied with each oxygen pulse from the oxygen source 20 to the passenger oxygen mask 12, may be adjusted as a function of the air pressure pₐᵢᵣ within the passenger cabin 104 in the normal breathing mode, in the fast breathing mode, in the slow breathing mode or in any two or all three of the normal breathing mode, the slow breathing mode, and the fast breathing mode.

The amount of oxygen V_{N}, V_{S}, V_{F}, which is supplied from the oxygen source 20 to the passenger oxygen mask 12, may be defined by an analytic function of the air pressure pₐᵢᵣ within the passenger cabin 104: V_{N/S/F} = g_{N/S/F} (pₐᵢᵣ). The same or different functions g_{N}, g_{S}, g_{F} may be applied for the normal breathing mode, for the slow breathing mode, and for the fast breathing mode, respectively.

Alternatively, the amount of oxygen V_{N}, V_{S}, V_{F}, which is supplied from the oxygen source 20 to the passenger oxygen mask 12, may be given as discrete values V_{N}(i), V_{S}(i), V_{F}(i), wherein a specific value V_{N}(i), V_{S}(i), V_{F}(i) is assigned to each of a plurality of ranges [p_{low}(i), p_{high}(i)] of air pressure pₐᵢᵣ within the passenger cabin 104. The borders p_{low}(i), p_{high}(i) of the ranges [p_{low}(i), p_{high}(i)] of air pressure pₐᵢᵣ and the discrete values V_{N}(i), V_{S}(i), V_{F}(i), which are assigned to the different ranges [p_{low}(i), p_{high}(i)] of air pressure pₐᵢᵣ, in the normal breathing mode may differ from the borders p_{low}(i), p_{high}(i) of the ranges [p_{low}(i), p_{high}(i)] of air pressure pₐᵢᵣ and the discrete values V_{N}(i), V_{S}(i), V_{F}(i), which are assigned to the different ranges [p_{low}(i), p_{high}(i)] of air pressure pₐᵢᵣ, in the in fast breathing mode and/or in the slow breathing mode.

The amounts of oxygen V_{N}, V_{S}, V_{F}, which are supplied from the oxygen source 20 to the passenger oxygen mask 12, may also be given by discrete values V_{N}(i), V_{S}(i), V_{F}(i) in at least one range [p_{low}(i), p_{high}(i)] of air pressure pₐᵢᵣ within the passenger cabin 104, and the amounts of oxygen V_{N}, V_{S}, V_{F} may be defined by a function g_{N/S/F}(pₐᵢᵣ) in at least one other range [p_{low}(k), p_{high}(k)] of air pressure pₐᵢᵣ within the passenger cabin 104.

The aircraft emergency oxygen supply system 2 may further comprise or may further be coupled to at least one temperature sensor 34, as depicted in Figure 3, which is configured for detecting the temperature in the environment of the aircraft emergency oxygen supply system 2, i.e. the temperature T in the aircraft passenger cabin 104.

In such an embodiment, the controller 18 may be configured for setting the amount of oxygen V_{N}, V_{S}, V_{F}, which is supplied to the passenger oxygen mask 12 at every breath of the passenger, as a function of the detected temperature T. This may apply to the normal breathing amount of oxygen V_{N}, which is provided in the normal breathing mode. This may also apply to the fast breathing amount of oxygen V_{F}, which is provided in the fast breathing mode, and/or to the slow breathing amount of oxygen V_{S}, which is provided in the slow breathing mode.

In order to compensate for a low supply of oxygen to the passenger during an initial time period between the loss of air pressure pₐᵢᵣ within the passenger cabin 104 and the supply of oxygen from the oxygen source 20 to the passenger via the passenger oxygen mask 12, a maximum amount of oxygen Vₘₐₓ may be supplied to the passenger oxygen mask 12 right after the start of the aircraft emergency oxygen supply system 2. The maximum amount of oxygen Vₘₐₓ may, for example, be supplied to the passenger oxygen mask 12 for the first 15 breaths taken by the passenger.

In other words, right after the start of the aircraft emergency oxygen supply system 2, the amount of oxygen V_{N}, V_{S}, V_{F}, which is supplied to the passenger oxygen mask 12 at each breath, may be set to the maximum value Vₘₐₓ independently of the air pressure pₐᵢᵣ within the passenger cabin 104.

Later, for example after 15 breaths have been taken by the passenger, the amount of oxygen V_{N}, V_{S}, V_{F}, which is supplied with each oxygen pulse from the oxygen source 20 to the passenger oxygen mask 12, may be adjusted as a function of the air pressure pₐᵢᵣ within the passenger cabin 104, as is has been described before.

The above described correspondence between the provision of bolus amounts of oxygen and bolus volumes of oxygen, which has been chosen for an easy comparison between the normal breathing mode, the fast breathing mode and the slow breathing mode, is based on the idealistic assumption that the working pressure of the oxygen, which may be supplied to the oxygen passenger mask via a controlled valve, is constant. In many realistic implementations, the working pressure may vary over the course of the emergency situation, e.g. due to the progressive emptying of the oxygen source and, potentially, other factors. Hence, in order to provide a desired bolus amount of oxygen to the passenger oxygen mask, i.e. to provide a desired bolus amount of oxygen in terms of oxygen molecules, different volumes of oxygen may be required, depending on the working pressure of the oxygen. This may be implemented via different opening times of the controlled valve, compensating for the variations in working pressure. It is stressed that the terminology of supplying a normal breathing amount of oxygen / a fast breathing amount of oxygen / a slow breathing amount of oxygen, as used herein, encompasses the adaptation of the bolus volume, depending on the working pressure and, potentially, other factors.

In an emergency situation, a drop of air pressure within the passenger cabin 104 may result in an insufficient supply of oxygen to the passenger, until the passenger manages to grab a passenger oxygen mask 12 and to put it over his/her face, in order to be supplied with oxygen form the oxygen source 20.

In order to avoid an undesirable switching into the fast breathing mode or into the slow breathing mode right after the start of the aircraft emergency oxygen supply system, the controller 18 may be configured not to switch into the fast breathing mode or into the slow breathing mode for a predetermined number of initial breaths, for example for the first 2 to 20 breaths, of the passenger, after the passenger oxygen mask 12 has been applied to his/her face and the supply of oxygen to the passenger has been started. In other words, the controller 18 may be configured for switching from the normal breathing mode into the fast breathing mode or into the slow breathing mode only if the breathing frequency indicator indicates a breathing frequency, which is above a predefined upper frequency threshold or which is below a predefined lower frequency threshold, after a predetermined number of breaths, for example between 2 and 20 breaths, in particular between 5 and 15 breaths, have been taken by the passenger.

The controller 18 may further be configured for determining the breathing frequency indicator from an average of a plurality of successive breath durations T(i) and switch into the fast breathing mode only if said average breathing frequency f(avg) indicates a fast breathing of the passenger, e.g. if the average breathing frequency f(avg) is above the predefined upper frequency threshold (f(avg) > f_{N}(high)). When expressed in terms of breath durations, the controller 18 may be configured to switch into the fast breathing mode only if the average breath duration T(avg) is below a predefined lower breath duration threshold (T(avg) < T_{N}(low)).

The controller 18 may also be configured for determining the breathing frequency indicator from an average of a plurality of successive breath durations T(i) and switch into the slow breathing mode only if said average breathing frequency f(avg) indicates a slow breathing of the passenger, e.g. if the average breathing frequency f(avg) is below the predefined lower frequency threshold (f(avg) < f_{N}(low)). When expressed in terms of breath durations, the controller 18 may be configured to switch into the slow breathing mode only if the average breath duration T(avg) is above a predefined upper breath duration threshold (T(avg) > T_{N}(high)).

The average may by the average over the last 5, 10, 15 or 20 detected breaths of the passenger. The average may by an arithmetic average or a geometric average. In order to achieve a high responsiveness of the aircraft emergency oxygen supply system and not to wait for the given numbers of detected breaths before reacting, the controller 18 may operate with an assumed breathing history at start-up. For example, it is possible that the controller 18, at start-up, assumes a breathing history of 5, 10, 15 or 20 breaths of the passenger with a set breathing duration, such as a breathing duration of 4 seconds, which corresponds to a breathing frequency of 15 breaths per minute. In this way, the controller 18 may, right from the start, determine the breathing frequency indicator from an average of a plurality of successive breath durations, while being able to provide a desired switching after a small number of breaths, such as 2 or 3 breaths, already.

The controller 18 may be configured for repeatedly determining the breathing frequency indicator on the basis of a moving average within a succession of breath durations T(i) of a succession of detected breaths of the passenger. Every average value of the moving average f(avg), T(avg) may be the result of an average of between 10 and 20 successive breath durations T(i), in particular the result of an average of between 12 and 18 successive breath durations T(i), further in particular the result of an average of 15 successive breath durations T(i).

The controller 18 may further be configured for applying a Kalman-Filter for damping accidental oscillations of the moving average f(avg), T(avg).

The controller 18 may be configured for switching into an enforced pulse mode if no breath of a passenger has been detected by the breath sensor 36 for a predefined period of time T_{enf}, referred to as breath enforce time T_{enf} herein.

The breath enforce time T_{enf} may be in the range of between 5 seconds and 15 seconds, the breath enforce time T_{enf} may in particular be in the range of between 9 seconds and 11 seconds, such as 10 seconds. After the controller 18 has been switched into the enforced pulse mode, the controller 18 may supply predefined amounts of oxygen Vₑₙₜ, which may be called enforced pulse amounts of oxygen V_{enf}, from the oxygen source 20 to the passenger oxygen mask 12 at predefined enforced mode time intervals ΔT_{enf}. In other words, when the controller 18 is operating in the enforced pulse mode, the supply of oxygen to the passenger oxygen mask is independent of the detection of passenger breaths by the breath sensor 36.

Operating the controller 18 in the enforced pulse mode may ensure that the passenger is supplied with a sufficient amount of oxygen even if his/her breaths are not detected by the breath sensor 36. It is possible that the breaths of the passenger are not detected by the breath sensor 36, because the passenger is not breathing strong enough and/or because the passenger oxygen mask 12 is not applied sufficiently tight to the passenger's face, which allows ambient air to flow uncontrolled into the passenger oxygen mask 12. In such a situation, the negative pressure, which is generated within the passenger oxygen mask 12, when the passenger inhales, is not sufficiently large for being detected as a breath by the breath sensor 36.

When operating in the enforced pulse mode, the enforced pulse amount of oxygen may be dependent on the ambient air pressure, i.e. the enforced pulse amount of oxygen may be a function of the ambient air pressure.

When operating in the enforced pulse mode, the controller 18 may be configured for supplying enforced pulse amounts of oxygen V_{enf} to the passenger oxygen mask 12 with a frequency f_{enf} of 40 to 60 pulses per minute, particularly with a frequency f_{enf} of 45 to 55 pulses per minute, more particularly with a frequency f_{enf} of 50 pulses per minute.

While the invention has been described with reference to exemplary embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed, but that the invention will include all embodiments falling within the scope of the appended claims.

## Claims

1. Aircraft emergency oxygen supply system (2) comprising:
a passenger oxygen mask (12) for supplying oxygen to a passenger wearing the passenger oxygen mask (12);
an oxygen source (20) for supplying oxygen to the passenger oxygen mask (12);
a breath sensor (36) for detecting breaths (30) of the passenger wearing the passenger oxygen mask (12); and
a controller (18) for controlling the supply of oxygen from the oxygen source (20) to the passenger oxygen mask (12);
wherein the controller (18) is configured for:
operating in a normal breathing mode, which includes supplying a normal breathing amount of oxygen (V_{N}) from the oxygen source (20) to the passenger oxygen mask (12) at every breath (30) of the passenger wearing the passenger oxygen mask (12);
deriving a breathing frequency indicator from a plurality of successive breaths (30) of the passenger; and
switching into a slow breathing mode if the breathing frequency indicator indicates a breathing frequency below a predefined lower frequency threshold, wherein operating in the slow breathing mode includes supplying a slow breathing amount of oxygen (V_{S}), which is larger than the normal breathing amount of oxygen (V_{N}), from the oxygen source (20) to the passenger oxygen mask (12) at every breath (30) of the passenger;
and/or
switching into a fast breathing mode if the breathing frequency indicator indicates a breathing frequency above a predefined upper frequency threshold, wherein operating in the fast breathing mode includes supplying a fast breathing amount of oxygen (V_{F}), which is smaller than the normal breathing amount of oxygen (V_{N}), from the oxygen source (20) to the passenger oxygen mask (12) at every breath (30) of the passenger.

2. Aircraft emergency oxygen supply system (2) according to claim 1,
wherein the predefined upper frequency threshold corresponds to a breathing frequency in the range of between 15 and 25 breaths (30) per minute, wherein the predefined upper frequency threshold corresponds in particular to a breathing frequency of 20 breaths (30) per minute;
and/or
wherein the predefined lower frequency threshold corresponds to a breathing frequency in the range of between 5 and 15 breaths (30) per minute, wherein the predefined lower frequency threshold corresponds in particular to a breathing frequency of 10 breaths (30) per minute.

3. Aircraft emergency oxygen supply system (2) according to claim 1 or 2,
wherein the plurality of successive breaths (30) of the passenger include a plurality of breath durations and wherein the controller (18) is configured for determining the breathing frequency indicator from an average of the plurality of breath durations,
wherein the controller (18) is in particular configured for repeatedly determining the breathing frequency indicator on the basis of a moving average within a succession of breath durations of a succession of breaths (30) of the passenger, with every average value of the moving average in particular being an average of between 10 and 20 breath durations, further in particular an average of 15 breath durations.

4. Aircraft emergency oxygen supply system (2) according to any of the preceding claims,
wherein the aircraft emergency oxygen supply system (2) comprises an ambient air pressure sensor (32), which is configured for detecting the pressure of air (pₐᵢᵣ) at or in the vicinity of the aircraft emergency oxygen supply system (2), wherein, in at least one of the normal breathing mode, the slow breathing mode, and the fast breathing mode, the amount of oxygen (V_{N}, V_{S}, V_{F}), which is supplied from the oxygen source (20) to the passenger oxygen mask (12), is a function of the pressure of air (pₐᵢᵣ) detected by the ambient air pressure sensor (32); and/or
wherein the aircraft emergency oxygen supply system (2) comprises a temperature sensor (34), which is configured for detecting the temperature (T) at or in the vicinity of the aircraft emergency oxygen supply system (2), wherein, in at least one of the normal breathing mode, the slow breathing mode, and the fast breathing mode, the amount of oxygen (V_{N}, V_{S}, V_{F}), which is supplied from the oxygen source (20) to the passenger oxygen mask (12), is a function of the temperature (T) detected by the temperature sensor (34).

5. Aircraft emergency oxygen supply system (2) according to claim 4, wherein, in at least one of the normal breathing mode, the slow breathing mode, and the fast breathing mode, the amount of oxygen (V_{N}, V_{S}, V_{F}), which is supplied from the oxygen source (20) to the passenger oxygen mask (12) at the beginning, in particular for the first 15 breaths of the passenger, is independent of the pressure of air (pₐᵢᵣ) detected by the ambient air pressure sensor (32).

6. Aircraft emergency oxygen supply system (2) according to any of the preceding claims,
wherein, in at least one of the normal breathing mode, the slow breathing mode, and the fast breathing mode, the amount of oxygen (V_{N}, V_{S}, V_{F}), which is supplied from the oxygen source (20) to the passenger oxygen mask (12), is a function of the breathing frequency indicator;
wherein, in at least one of the normal breathing mode, the slow breathing mode, and the fast breathing mode, the amount of oxygen (V_{N}, V_{S}, V_{F}), which is supplied from the oxygen source (20) to the passenger oxygen mask (12), is in particular inversely proportional to the breathing frequency indicated by the breathing frequency indicator.

7. Aircraft emergency oxygen supply system (2) according to any of the preceding claims,
wherein the controller (18) is configured for operating in an enforced pulse mode if no breath (30) of a passenger has been detected by the breath sensor (36) for a predefined breath enforce time (Tₑₙₜ), wherein operating in the enforced pulse mode includes supplying predefined amounts of oxygen (V_{enf}) from the oxygen source (20) to the passenger oxygen mask (12) at predefined time intervals (ΔT_{enf});
wherein the predefined breath enforce time (T_{enf}), in which no breath (30) has been detected, is in particular in the range of between 5 seconds and 15 seconds, wherein the predefined breath enforce time (T_{enf}) is more particularly in the range of between 9 seconds and 11 seconds; and/or
wherein operating in the enforced pulse mode includes in particular supplying predefined amounts (V_{enf}) of oxygen from the oxygen source (20) to the passenger oxygen mask (12) with a frequency of 40 to 60 pulses per minute, more particularly with a frequency of 45 to 55 pulses per minute.

8. Aircraft emergency oxygen supply system (2) according to any of the preceding claims, wherein the aircraft emergency oxygen supply system (2) comprises a plurality of passenger oxygen masks (12), and wherein the controller (18) is configured for individually controlling the supply of oxygen to each of the plurality of passenger oxygen masks (12);
wherein the aircraft emergency oxygen supply system (2) comprises in particular two, three, four, five or six passenger oxygen masks (12).

9. Passenger aircraft (100), in particular an airplane or a helicopter, comprising at least one aircraft emergency oxygen supply system (2) according to any of the preceding claims.

10. Method of supplying oxygen to a passenger oxygen mask (12) in an aircraft (100), wherein the method includes:
detecting breaths (30) of a passenger wearing the passenger oxygen mask (12);
operating in a normal breathing mode, which includes supplying a normal breathing amount of oxygen (V_{N}) from the oxygen source (20) to the passenger oxygen mask (12) at every breath (30) of the passenger wearing the passenger oxygen mask (12);
deriving a breathing frequency indicator from a plurality of successive breaths (30) of the passenger; and
switching into a slow breathing mode if the breathing frequency indicator indicates a breathing frequency below a predefined lower frequency threshold, wherein operating in the slow breathing mode includes supplying a slow breathing amount of oxygen (V_{S}), which is larger than the normal breathing amount of oxygen (V_{N}), from the oxygen source (20) to the passenger oxygen mask (12) at every breath (30) of the passenger;
and/or
switching into a fast breathing mode if the breathing frequency indicator indicates a breathing frequency above a predefined upper frequency threshold, wherein operating in the fast breathing mode includes supplying a fast breathing amount of oxygen (V_{F}), which is smaller than the normal breathing amount of oxygen (V_{N}), from the oxygen source (20) to the passenger oxygen mask (12) at every breath (30) of the passenger.

11. Method according to claim 10,
wherein the predefined upper frequency threshold corresponds to a breathing frequency in the range of between 15 and 25 breaths (30) per minute, wherein the predefined upper frequency threshold corresponds in particular to a breathing frequency of 20 breaths (30) per minute;
and/or
wherein the predefined lower frequency threshold corresponds to a breathing frequency in the range of between 5 and 15 breaths (30) per minute, wherein the predefined lower frequency threshold corresponds in particular to a breathing frequency of 10 breaths (30) per minute.

12. Method according to claim 10 or 11,
wherein the plurality of successive breaths (30) of the passenger include a plurality of breath durations and wherein the method includes determining the breathing frequency indicator from an average of the plurality of breath durations;
wherein the method includes in particular repeatedly determining the breathing frequency indicator on the basis of a moving average within a succession of breath durations of a succession of breaths (30) of the passenger, with every average value of the moving average in particular being an average of between 10 and 20 breath durations, further in particular an average of 15 breath durations.

13. Method according to any of claims 10 to 12,
wherein, in at least one of the normal breathing mode, the slow breathing mode and the fast breathing mode, the amount of oxygen (V_{N}, V_{S}, V_{F}), which is supplied from the oxygen source (20) to the passenger oxygen mask (12), is a function of the breathing frequency indicator, wherein the amount of oxygen (V_{N}, V_{S}, V_{F}), which is supplied from the oxygen source (20) to the passenger oxygen mask (12), is in particular inversely proportional to the breathing frequency indicated by the breathing frequency indicator; and/or
wherein, in at least one of the normal breathing mode, the slow breathing mode and the fast breathing mode, the amount of oxygen (V_{N}, V_{S}, V_{F}), which is supplied from the oxygen source (20) to the passenger oxygen mask (12), is independent of the breathing frequency indicated by the breathing frequency indicator.

14. Method according to any of claims 10 to 13, wherein the method includes detecting an ambient air pressure (pₐᵢᵣ) and/or a temperature (T) at the aircraft emergency oxygen supply system (2), and wherein, in at least one of the normal breathing mode, the slow breathing mode, and the fast breathing mode, the amount of oxygen (V_{N}, V_{S}, V_{F}), which is supplied from the oxygen source (20) to the passenger oxygen mask (12), is a function of the detected ambient air pressure (pₐᵢᵣ) and/or of the detected temperature (T).

15. Method according to any of claims 10 to 14, wherein the method includes operating in an enforced pulse mode if no breath (30) of a passenger has been detected by the breath sensor (36) for a predefined breath enforce time (T_{enf}), wherein operating in the enforced pulse mode includes supplying predefined amounts of oxygen (V_{enf}) from the oxygen source (20) to the passenger oxygen mask (12) at predefined time intervals (ΔT_{enf});
wherein the predefined breath enforce time (T_{enf}), in which no breath (30) has been detected, is in particular in the range of between 5 seconds and 15 seconds, wherein the predefined time period is more particularly in the range of between 9 seconds and 11 seconds; and/or
wherein operating in the enforced pulse mode includes in particular supplying predefined amounts (V_{enf}) of oxygen from the oxygen source (20) to the passenger oxygen mask (12) with a frequency of 40 to 60 pulses per minute, more particularly with a frequency of 45 to 55 pulses per minute.
